# EUROPEAN PATENT SPECIFICATION

(11) **EP 1 443 117 B1**
(45) Date of publication and mention of the grant of the patent: **11.08.2010**
(21) Application number: 03018045.9
(22) Date of filing: 09.11.2001
(51) Int. Cl.: C12Q 1/48, C12N 9/12, C12N 15/00, C12N 1/20, C07K 1/00

(54) **Human testis specific serine/threonine kinase**
Menschliche hodenspezifische Serin/Threonin-Kinase
Serine/thréonine kinase spécifique des testicules de l'homme

(30) Priority: 09.11.2000 US 246939 P; 30.01.2001 US 264921 P
(43) Date of publication of application: 04.08.2004
(62) Divisional of application: 01986508.8
(73) Proprietor: UNIVERSITY OF VIRGINIA PATENT FOUNDATION, Charlottesville, Virginia 22903 (US); Kopf, Gregory S., Wynnewood, PA 19096 (US)
(72) Inventor: Herr, John C., Charlottsville, VA 22901 (US); Visconti, Pablo, Amherst, MA 01002 (US); Hao, Zhonglin, Charlottesville, VA 22911 (US); Kopf, Gregory S., Wynnewood, PA 19096 (US)
(74) Representative: Graf von Stosch, Andreas

(56) References cited:
- WO-A2-00/73469
- WO-A2-01/85954
- VISCONTI P E ET AL: "Cloning and Chromosomal Localization of a Gene Encoding a Novel Serine/Threonine Kinase Belonging to the Subfamily of Testis-Specific Kinases" GENOMICS, ACADEMIC PRESS, SAN DIEGO, US, vol. 77, no. 3, October 2001 (2001-10), pages 163-170, XP004468720 ISSN: 0888-7543
- ZUERCHER GISELA ET AL: "A novel member of the testis specific serine kinase family, tssk-3, expressed in the Leydig cells of sexually mature mice" MECHANISMS OF DEVELOPMENT, ELSEVIER SCIENCE IRELAND LTD, IE, vol. 93, no. 1-2, May 2000 (2000-05), pages 175-177, XP002182408 ISSN: 0925-4773
- TOSHIMA JIRO ET AL: "Identification and characterization of a novel protein kinase, TESK1, specifically expressed in testicular germ cells" JOURNAL OF BIOLOGICAL CHEMISTRY, vol. 270, no. 52, 1995, pages 31331-31337, XP002308008 ISSN: 0021-9258
- KUENG P ET AL: "A novel family of serine/threonine kinases participating in spermiogenesis" THE JOURNAL OF CELL BIOLOGY, ROCKEFELLER UNIVERSITY PRESS, US, vol. 139, no. 7, 29 December 1997 (1997-12-29), pages 1851-1859, XP002168387 ISSN: 0021-9525
- "Faculty / Core Leaders "John Herr"" UNIVERSITY OF VIRGINIA, 18 October 2000 (2000-10-18), pages 1-2, XP002308009 Retrieved from the Internet: URL:http://web.archive.org/web/20000918171 959/hsc.virginia.edu/medicine/basic-sci/ce llbio/crgcv/people/jch7k.html>
- VISCONTI P E ET AL: "Cholesterol efflux-mediated signal transduction in mammalian sperm: cholesterol release signals an increase in protein tyrosine phosphorylation during mouse sperm capacitation." DEVELOPMENTAL BIOLOGY. 15 OCT 1999, vol. 214, no. 2, 15 October 1999 (1999-10-15), pages 429-443, XP002308010 ISSN: 0012-1606
- DE LAMIRANDE E ET AL: "Capacitation as a regulatory event that primes spermatozoa for the acrosome reaction and fertilization." MOLECULAR HUMAN REPRODUCTION. MAR 1997, vol. 3, no. 3, March 1997 (1997-03), pages 175-194, XP002308011 ISSN: 1360-9947
- DATABASE EMBL [Online] 17 August 1999 (1999-08-17), "Human DNA sequence from clone RP4-811H24 on chromosome 1p34.1-34.3 Contains the 3' end of LCK gene for lymphocyte-specific protein tyrosine kinase, HDAC1 gene for histone deacetylase 1, the MLP gene for MARCKS-like protein, STK22C gene for serine/threonine kinase 22C (spermiogenesis associated), a n" XP002308012 retrieved from EBI accession no. EM_PRO:HSJ811H24 Database accession no. HSJ811H24
- DATABASE EMBL [Online] 17 September 1999 (1999-09-17), "Homo sapiens chromosome 5 clone CTD-2201G3, complete sequence." XP002308771 retrieved from EBI accession no. EM_PRO:AC010431 Database accession no. AC010431
- DATABASE EMBL [Online] 18 June 2000 (2000-06-18), "Human DNA sequence *** SEQUENCING CANCELLED *** from clone RP13-67O19" XP002308772 retrieved from EBI accession no. EM_PRO:AL359537 Database accession no. AL359537
- DATABASE EMBL [Online] 17 August 1999 (1999-08-17), "Human DNA sequence from clone RP4-811H24 on chromosome 1p34.1-34.3 Contains the 3' end of LCK gene for lymphocyte-specific protein tyrosine kinase, HDAC1 gene for histone deacetylase 1, the MLP gene for MARCKS-like protein, STK22C gene for serine/threonine kinase 22C (spermiogenesis associated), a n" XP002308773 retrieved from EBI accession no. EM_PRO:HSJ811H24 Database accession no. HSJ811H24

### SEQUENCE LISTING

<110> Herr, John
   Kopf, Gregory
   Visconti, Pablo
   Hao, Zhonglin
<120> Human Testis Specific Serine/Threonine Kinase
<130> 00623-03
<150> US 60/246,939
<151> 2000-11-09
<150> US 60/264,921
   <151> 2001-01-30
<160> 18
<170> PatentIn version 3.0
<210> 1
   <211> 1352
   <212> DNA
   <213> Homo sapiens
<400> 1
   tctatccagg atgtaaatga gcacactgct ggcccatgcg cctcggggct gtagagggca   60
   gcctcagagg cactgggcat tcctggcacc atggatgacg ctgctgtcct caagcgacga  120
   ggctacctcc tggggataaa tttaggagag ggctcctatg caaaagtaaa atctgcttac  180
   tctgagcgcc tgaagttcaa tgtggcgatc aagatcatcg accgcaagaa ggcccccgca  240
   gacttcttgg agaaattcct tccccgggaa attgagattc tggccatgtt aaaccactgc  300
   tccatcatta agacctacga gatctttgag acatcacatg gcaaggtcta catcgtcatg  360
   gagctcgcgg tccagggcga cctcctcgag ttaatcaaaa cccggggagc cctgcatgag  420
   gacgaagctc gcaagaagtt ccaccagctt tccttggcca tcaagtactg ccacgacctg  480
   gacgtcgtcc accgggacct caagtgtgac aaccttctcc ttgacaagga cttcaacatc  540
   aagctgtccg acttcagctt ctccaagcgc tgcctgcggg atgacagtgg tcgaatggca  600
   ttaagcaaga ccttctgtgg gtcaccagcg tatgcggccc cagaggtgct gcagggcatt  660
   ccctaccagc ccaaggtgta cgacatctgg agcctaggcg tgatcctcta catcatggtc  720
   tgcggctcca tgccctacga cgactccaac atcaagaaga tgctgcgtat ccagaaggag  780
   caccgcgtca acttcccacg ctccaagcac ctgacaggcg agtgcaagga cctcatctac  840
   cacatgctgc agcccgacgt caaccggcgg ctccacatcg acgagatcct cagccactgc  900
   tggatgcagc ccaaggcacg gggatctccc tctgtggcca tcaacaagga gggggagagt  960
   tcccggggaa ctgaaccctt gtggaccccc gaacctggct ctgacaagaa gtctgccacc 1020
   aagctggagc ctgagggaga ggcacagccc caggcacagc ctgagacaaa acccgagggg 1080
   acagcaatgc aaatgtccag gcagtcggag atcctgggtt tccccagcaa gccgtcgact 1140
   atggagacag aggaagggcc cccccaacag cctccagaga cgcgggccca gtgagcttct 1200
   tgcggcccag ggaatgagat ggagctcacg gcttaaagcc caagctctga agaagtcaag 1260
   ggtggagcca gagaaggaag gcagtcccag atgagcctct attttcatca gcttcttctc 1320
   tctccccttg aacttggtaa cccacatggt tc                               1352
<210> 2
   <211> 1254
   <212> DNA
   <213> Homo sapiens
<400> 2
   ttgaggacaa cgcctgctgg cccacatgac ggggggatgt agacggcagc ggcgccagtc   60
   gctcctggca ccatggacga tgccacagtc ctaaggaaga agggttacat cgtaggcatc  120
   aatcttggca agggttccta cgcaaaagtc aaatctgcct actctgagcg cctcaagttc  180
   aatgtggctg tcaagatcat cgaccgcaag aaaacaccta ctgactttgt ggagagattc  240
   cttcctcggg agatggacat cctggcaact gtcaaccacg gctccatcat caagacttac  300
   gagatctttg agacctctga cggacggatc tacatcatca tggagcttgg cgtccagggc  360
   gacctcctcg agttcatcaa gtgccaggga gccctgcatg aggacgtggc acgcaagatg  420
   ttccgacagc tctcctccgc cgtcaagtac tgccacgacc tggacatcgt ccaccgggac  480
   ctcaagtgcg agaaccttct cctcgacaag gacttcaaca tcaagctgtc tgactttggc  540
   ttctccaagc gctgcctgcg ggacagcaat gggcgcatca tcctcagcaa gaccttctgc  600
   gggtcggcag catatgcagc ccccgaggtg ctgcagagca tcccctacca gcccaaggtg  660
   tatgacatct ggagcctggg cgtgatcctg tacatcatgg tctgcggctc catgccctat  720
   gacgactccg acatcaggaa gatgctgcgt atccagaagg agcaccgtgt ggacttcccg  780
   cgctccaaga acctgacctg cgagtgcaag gacctcatct accgcatgct gcagcccgac  840
   gtcagccagc ggctccacat cgatgagatc ctcagccact cgtggctgca gccccccaag  900
   cccaagccaa cgtcttctgc ttccttcaag agggaggggg agggcaagta ccgcgctgag  960
   tgcaaactgg acaccaagac aggcttgagg cccgaccacc ggcccgacca caagcttgga 1020
   gccaaaaccc agcaccggct gctggtggtg cccgagaacg agaacaggat ggaggacagg 1080
   ctggccgaga cctccagggc caaagaccat cacatctccg gagctgaggt ggggaaagca 1140
   agcacctagc atgacaatgg ccccgttgtg tgtggtgggg gtcggggttg gggggcatgg 1200
   tgcagtcggc cttcacgtaa actaagtagg caggtaggat ctgaagaagg caca        1254
<210> 3
   <211> 1058
   <212> DNA
   <213> Homo sapiens
<400> 3
   catcctaata cgactcacta tagggctcga gcggcgcccg ggcaggtgag aatgttctaa   60
   cgctgggggc ggctgcggat gaagtccttg gggagaaaag gagcaggcca agggcgatgg  120
   tggagtagag ctgcctctca gaggcagcat gagctgagag ggtgatagga aggcggcgct  180
   agacagcatg gaggactttc tgctctccaa tgggtaccag ctgggcaaga ccattgggga  240
   agggacctac tcaaaagtca aagaagcatt ttccaaaaaa caccaaagaa aagtggcaat  300
   taaagttata gacaagatgg gagggccaga agagtttatc cagagattcc tccctcggga  360
   gctccaaatc gtccgtaccc tggaccacaa gaacatcatc caggtgtatg agatgctgga  420
   gtctgccgac gggaaaatct gcctggtgat ggagctcgct gagggagggg atgtctttga  480
   ctgcgtgctg aatggggggc cactgcctga aagccgggcc aaggccctct tccgtcagat  540
   ggttgaggcc atccgctact gccatggctg tggtgtggcc caccgggacc tcaaatgtga  600
   gaacgccttg ttgcagggct tcaacctgaa gctgactgac tttggctttg ccaaggtgtt  660
   gcccaagtca caccgggagc tgagccagac cttctgcggc agtacagcct atgctgcccc  720
   cgaggtgctg cagggcattc cccacgatag caaaaaaggt gatgtctgga gcatgggtgt  780
   ggtcctgtat gtcatgctct gtgccagcct accttttgac gacacagaca tccccaagat  840
   gctgtggcag cagcagaagg gggtgtcctt ccccactcat ctgagcatct cggccgattg  900
   ccaggacctg ctcaagaggc tcctggaacc cgatatgatc ctccggcctt caattgaaga  960
   agttagttgg catccatggc tagcaagcac ttgataaaag caatggcaag tgctctccaa 1020
   taaagtaggg ggagaaagca aagcaaaccc aaaaaaaa                         1058
<210> 4
   <211> 367
   <212> PRT
   <213> Homo sapiens
<400> 4
   Met Asp Asp Ala Ala Val Leu Lys Arg Arg Gly Tyr Leu Leu Gly Ile
   1               5                   10                  15
   Asn Leu Gly Glu Gly Ser Tyr Ala Lys Val Lys Ser Ala Tyr Ser Glu
               20                  25                  30
   Arg Leu Lys Phe Asn Val Ala Ile Lys Ile Ile Asp Arg Lys Lys Ala
           35                  40                  45
   Pro Ala Asp Phe Leu Glu Lys Phe Leu Pro Arg Glu Ile Glu Ile Leu
       50                  55                  60
   Ala Met Leu Asn His Cys Ser Ile Ile Lys Thr Tyr Glu Ile Phe Glu
   65                  70                  75                  80
   Thr Ser His Gly Lys Val Tyr Ile Val Met Glu Leu Ala Val Gln Gly
                   85                  90                  95
   Asp Leu Leu Glu Leu Ile Lys Thr Arg Gly Ala Leu His Glu Asp Glu
               100                 105                 110
   Ala Arg Lys Lys Phe His Gln Leu Ser Leu Ala Ile Lys Tyr Cys His
           115                 120                 125
   Asp Leu Asp Val Val His Arg Asp Leu Lys Cys Asp Asn Leu Leu Leu
       130                 135                 140
   Asp Lys Asp Phe Asn Ile Lys Leu Ser Asp Phe Ser Phe Ser Lys Arg
   145                 150                 155                 160
   Cys Leu Arg Asp Asp Ser Gly Arg Met Ala Leu Ser Lys Thr Phe Cys
                   165                 170                 175
   Gly Ser Pro Ala Tyr Ala Ala Pro Glu Val Leu Gln Gly Ile Pro Tyr
               180                 185                 190
   Gln Pro Lys Val Tyr Asp Ile Trp Ser Leu Gly Val Ile Leu Tyr Ile
   195 200 205
   Met Val Cys Gly Ser Met Pro Tyr Asp Asp Ser Asn Ile Lys Lys Met
   210 215 220
   Leu Arg Ile Gln Lys Glu His Arg Val Asn Phe Pro Arg Ser Lys His
   225 230 235 240
   Leu Thr Gly Glu Cys Lys Asp Leu Ile Tyr His Met Leu Gln Pro Asp
   245 250 255
   Val Asn Arg Arg Leu His Ile Asp Glu Ile Leu Ser His Cys Trp Met
   260 265 270
   Gln Pro Lys Ala Arg Gly Ser Pro Ser Val Ala Ile Asn Lys Glu Gly
   275 280 285
   Glu Ser Ser Arg Gly Thr Glu Pro Leu Trp Thr Pro Glu Pro Gly Ser
   290 295 300
   Asp Lys Lys Ser Ala Thr Lys Leu Glu Pro Glu Gly Glu Ala Gln Pro
   305 310 315 320
   Gln Ala Gln Pro Glu Thr Lys Pro Glu Gly Thr Ala Met Gln Met Ser
   325 330 335
   Arg Gln Ser Glu Ile Leu Gly Phe Pro Ser Lys Pro Ser Thr Met Glu
   340 345 350
   Thr Glu Glu Gly Pro Pro Gln Gln Pro Pro Glu Thr Arg Ala Gln
   355 360 365
<210> 5
   <211> 358
   <212> PRT
   <213> Homo sapiens
<400> 5
   Met Asp Asp Ala Thr Val Leu Arg Lys Lys Gly Tyr Ile Val Gly Ile
   1 5 10 15
   Asn Leu Gly Lys Gly Ser Tyr Ala Lys Val Lys Ser Ala Tyr Ser Glu
   20 25 30
   Arg Leu Lys Phe Asn Val Ala Val Lys Ile Ile Asp Arg Lys Lys Thr
   35 40 45
   Pro Thr Asp Phe Val Glu Arg Phe Leu Pro Arg Glu Met Asp Ile Leu
   50 55 60
   Ala Thr Val Asn His Gly Ser Ile Ile Lys Thr Tyr Glu Ile Phe Glu
   65 70 75 80
   Thr Ser Asp Gly Arg Ile Tyr Ile Ile Met Glu Leu Gly Val Gln Gly
   85 90 95
   Asp Leu Leu Glu Phe Ile Lys Cys Gln Gly Ala Leu His Glu Asp Val
   100 105 110
   Ala Arg Lys Met Phe Arg Gln Leu Ser Ser Ala Val Lys Tyr Cys His
   115 120 125
   Asp Leu Asp Ile Val His Arg Asp Leu Lys Cys Glu Asn Leu Leu Leu
   130 135 140
   Asp Lys Asp Phe Asn Ile Lys Leu Ser Asp Phe Gly Phe Ser Lys Arg
   145 150 155 160
   Cys Leu Arg Asp Ser Asn Gly Arg Ile Ile Leu Ser Lys Thr Phe Cys
   165 170 175
   Gly Ser Ala Ala Tyr Ala Ala Pro Glu Val Leu Gln Ser Ile Pro Tyr
   180 185 190
   Gln Pro Lys Val Tyr Asp Ile Trp Ser Leu Gly Val Ile Leu Tyr Ile
   195 200 205
   Met Val Cys Gly Ser Met Pro Tyr Asp Asp Ser Asp Ile Arg Lys Met
   210 215 220
   Leu Arg Ile Gln Lys Glu His Arg Val Asp Phe Pro Arg Ser Lys Asn
   225 230 235 240
   Leu Thr Cys Glu Cys Lys Asp Leu Ile Tyr Arg Met Leu Gln Pro Asp
   245 250 255
   Val Ser Gln Arg Leu His Ile Asp Glu Ile Leu Ser His Ser Trp Leu
   260 265 270
   Gln Pro Pro Lys Pro Lys Ala Thr Ser Ser Ala Ser Phe Lys Arg Glu
   275 280 285
   Gly Glu Gly Lys Tyr Arg Ala Glu Cys Lys Leu Asp Thr Lys Thr Gly
   290 295 300
   Leu Arg Pro Asp His Arg Pro Asp His Lys Leu Gly Ala Lys Thr Gln
   305 310 315 320
   His Arg Leu Leu Val Val Pro Glu Asn Glu Asn Arg Met Glu Asp Arg
   325 330 335
   Leu Ala Glu Thr Ser Arg Ala Lys Asp His His Ile Ser Gly Ala Glu
   340 345 350
   Val Gly Lys Ala Ser Thr
   355
<210> 6
   <211> 268
   <212> PRT
   <213> Homo sapiens
<400> 6
   Met Glu Asp Phe Leu Leu Ser Asn Gly Tyr Gln Leu Gly Lys Thr Ile
   1 5 10 15
   Gly Glu Gly Thr Tyr Ser Lys Val Lys Glu Ala Phe Ser Lys Lys His
   20 25 30
   Gln Arg Lys Val Ala Ile Lys Val Ile Asp Lys Met Gly Gly Pro Glu
   35 40 45
   Glu Phe Ile Gln Arg Phe Leu Pro Arg Glu Leu Gln Ile Val Arg Thr
   50 55 60
   Leu Asp His Lys Asn Ile Ile Gln Val Tyr Glu Met Leu Glu Ser Ala
   65 70 75 80
   Asp Gly Lys Ile Cys Leu Val Met Glu Leu Ala Glu Gly Gly Asp Val
   85 90 95
   Phe Asp Cys Val Leu Asn Gly Gly Pro Leu Pro Glu Ser Arg Ala Lys
   100 105 110
   Ala Leu Phe Arg Gln Met Val Glu Ala Ile Arg Tyr Cys His Gly Cys
   115 120 125
   Gly Val Ala His Arg Asp Leu Lys Cys Glu Asn Ala Leu Leu Gln Gly
   130 135 140
   Phe Asn Leu Lys Leu Thr Asp Phe Gly Phe Ala Lys Val Leu Pro Lys
   145 150 155 160
   Ser His Arg Glu Leu Ser Gln Thr Phe Cys Gly Ser Thr Ala Tyr Ala
   165 170 175
   Ala Pro Glu Val Leu Gln Gly Ile Pro His Asp Ser Lys Lys Gly Asp
   180 185 190
   Val Trp Ser Met Gly Val Val Leu Tyr Val Met Leu Cys Ala Ser Leu
   195 200 205
   Pro Phe Asp Asp Thr Asp Ile Pro Lys Met Leu Trp Gln Gln Gln Lys
   210 215 220
   Gly Val Ser Phe Pro Thr His Leu Ser Ile Ser Ala Asp Cys Gln Asp
   225 230 235 240
   Leu Leu Lys Arg Leu Leu Glu Pro Asp Met Ile Leu Arg Pro Ser Ile
   245 250 255
   Glu Glu Val Ser Trp His Pro Trp Leu Ala Ser Thr
   260 265
<210> 7
   <211> 1779
   <212> DNA
   <213> Homo sapiens
<400> 7
   atggcgagcg tggtggtgaa gacgatctgg cagtccaaag agatccatga ggccggggac 60
   acccccacgg gggtggagag ctgctcccag ctagtcccag aggctccccg gagggtgacc 120
   agccgggcca aggggatccc gaagaaaaag aaggccgtgt cgttccacgg ggtggagccc 180
   cagatgtccc atcagcccat gcactggtgc ctgaacctca aacggtcctc ggcctgcacc 240
   aacgtgtcac tgctcaacct ggccgccatg gagcccactg actccacggg gacagactcc 300
   acagtggaag acctcagcgg ccaactcaca ctggctgggc cccctgcctc ccctacccta 360
   ccctgggatc cggatgacgc agacatcacg gaaatcctga gtggggtcaa cagtggattg 420
   gtccgcgcca aagactccat caccagcttg aaggaaaaga ccaaccgggt taaccagcac 480
   gtgcagtctc tgcagagcga gtgttctgtg ctgagcgaga atctggaggg aaggcggcaa 540
   gaggcagaag agttggaggg gtactgcatt caactcaagg agaactgctg gaaggtgacc 600
   cggtctgtgg aagatgctga aatcaaaacc aacgtcttga agcagaattc tgccctgctg 660
   gaggagaagt tgcgctacct ccagcagcag ctgcaggatg agacgccgcg acggcaggag 720
   gccgagctgc aggagccgga ggagaagcag gagccggagg agaagcagga gccggaggag 780
   aagcagaagc cggaggctgg cctctcctgg aacagcctgg gccccgccgc cacgtcccag 840
   ggctgccccg gcccgccagg gagtcccgac aaaccctcgc ggccacacgg cctggtcccc 900
   gcaggctggg gaatggggcc tcgggctggc gagggcccct acgtgagcga gcaggaattg 960
   cagaagctgt tcaccggcat cgaagagctg aggagagagg tgtcctcact gaccgcccgg 1020
   tggcatcagg aggagggggc ggtgcaggaa gccctgcggc tgctcggggg cctgggcggc 1080
   agggtcgacg gcttcctagg ccagtgggag cgggcacagc gcgaacaggc acagacggcg 1140
   cggggcttgc aggagctgcg aggtcgggcg gatgagctgt gcaccatggt ggagcggtca 1200
   gcagtgtctg tggcttcact gaggagcgaa ctggaggggc tgggcccact gaaacccatt 1260
   ctggaggagt tcgggcggca atttcagaac tctcgaagag ggcctgacct ctccatgaac 1320
   ctggatcggt cccaccaagg caactgtgcc cgctgtgcca gccaggggtc gcagttgtct 1380
   acggagtccc tgcagcagct gctggaccga gcactgacct cactagtgga cgaggtgaag 1440
   cagaggggcc tgactcctgc ctgtcccagc tgtcagaggc tacacaagaa gattctggag 1500
   ctggagcgcc aggccttagc caaacacgtc agggcagagg ccctgagctc cacccttcgg 1560
   ctggcccaag acgaggccct gcgggccaag aacctactgc tgacagacaa gatgaagcca 1620
   gaggagaaga tggccactct ggaccatcta cacttgaaga tgtgctccct ccacgatcat 1680
   ctcagcaacc tgccacttga ggggtccacg ggaacaatgg ggggaggcag cagtgcagga 1740
   acccccccaa aacagggggg ctcagcccct gaacaataa 1779
<210> 8
   <211> 592
   <212> PRT
   <213> Homo sapiens
<400> 8
   Met Ala Ser Val Val Val Lys Thr Ile Trp Gln Ser Lys Glu Ile His
   1 5 10 15
   Glu Ala Gly Asp Thr Pro Thr Gly Val Glu Ser Cys Ser Gln Leu Val
   20 25 30
   Pro Glu Ala Pro Arg Arg Val Thr Ser Arg Ala Lys Gly Ile Pro Lys
   35 40 45
   Lys Lys Lys Ala Val Ser Phe His Gly Val Glu Pro Gln Met Ser His
   50 55 60
   Gln Pro Met His Trp Cys Leu Asn Leu Lys Arg Ser Ser Ala Cys Thr
   65 70 75 80
   Asn Val Ser Leu Leu Asn Leu Ala Ala Met Glu Pro Thr Asp Ser Thr
   85 90 95
   Gly Thr Asp Ser Thr Val Glu Asp Leu Ser Gly Gln Leu Thr Leu Ala
   100 105 110
   Gly Pro Pro Ala Ser Pro Thr Leu Pro Trp Asp Pro Asp Asp Ala Asp
   115 120 125
   Ile Thr Glu Ile Leu Ser Gly Val Asn Ser Gly Leu Val Arg Ala Lys
   130 135 140
   Asp Ser Ile Thr Ser Leu Lys Glu Lys Thr Asn Arg Val Asn Gln His
   145 150 155 160
   Val Gln Ser Leu Gln Ser Glu Cys Ser Val Leu Ser Glu Asn Leu Glu
   165 170 175
   Gly Arg Arg Gln Glu Ala Glu Glu Leu Glu Gly Tyr Cys Ile Gln Leu
   180 185 190
   Lys Glu Asn Cys Trp Lys Val Thr Arg Ser Val Glu Asp Ala Glu Ile
   195 200 205
   Lys Thr Asn Val Leu Lys Gln Asn Ser Ala Leu Leu Glu Glu Lys Leu
   210 215 220
   Arg Tyr Leu Gln Gln Gln Leu Gln Asp Glu Thr Pro Arg Arg Gln Glu
   225 230 235 240
   Ala Glu Leu Gln Glu Pro Glu Glu Lys Gln Glu Pro Glu Glu Lys Gln
   245 250 255
   Glu Pro Glu Glu Lys Gln Lys Pro Glu Ala Gly Leu Ser Trp Asn Ser
   260 265 270
   Leu Gly Pro Ala Ala Thr Ser Gln Gly Cys Pro Gly Pro Pro Gly Ser
   275 280 285
   Pro Asp Lys Pro Ser Arg Pro His Gly Leu Val Pro Ala Gly Trp Gly
   290 295 300
   Met Gly Pro Arg Ala Gly Glu Gly Pro Tyr Val Ser Glu Gln Glu Leu
   305 310 315 320
   Gln Lys Leu Phe Thr Gly Ile Glu Glu Leu Arg Arg Glu Val Ser Ser
   325 330 335
   Leu Thr Ala Arg Trp His Gln Glu Glu Gly Ala Val Gln Glu Ala Leu
   340 345 350
   Arg Leu Leu Gly Gly Leu Gly Gly Arg Val Asp Gly Phe Leu Gly Gln
   355 360 365
   Trp Glu Arg Ala Gln Arg Glu Gln Ala Gln Thr Ala Arg Gly Leu Gln
   370 375 380
   Glu Leu Arg Gly Arg Ala Asp Glu Leu Cys Thr Met Val Glu Arg Ser
   385 390 395 400
   Ala Val Ser Val Ala Ser Leu Arg Ser Glu Leu Glu Gly Leu Gly Pro
   405 410 415
   Leu Lys Pro Ile Leu Glu Glu Phe Gly Arg Gln Phe Gln Asn Ser Arg
   420 425 430
   Arg Gly Pro Asp Leu Ser Met Asn Leu Asp Arg Ser His Gln Gly Asn
   435 440 445
   Cys Ala Arg Cys Ala Ser Gln Gly Ser Gln Leu Ser Thr Glu Ser Leu
   450 455 460
   Gln Gln Leu Leu Asp Arg Ala Leu Thr Ser Leu Val Asp Glu Val Lys
   465 470 475 480
   Gln Arg Gly Leu Thr Pro Ala Cys Pro Ser Cys Gln Arg Leu His Lys
   485 490 495
   Lys Ile Leu Glu Leu Glu Arg Gln Ala Leu Ala Lys His Val Arg Ala
   500 505 510
   Glu Ala Leu Ser Ser Thr Leu Arg Leu Ala Gln Asp Glu Ala Leu Arg
   515 520 525
   Ala Lys Asn Leu Leu Leu Thr Asp Lys Met Lys Pro Glu Glu Lys Met
   530 535 540
   Ala Thr Leu Asp His Leu His Leu Lys Met Cys Ser Leu His Asp His
   545 550 555 560
   Leu Ser Asn Leu Pro Leu Glu Gly Ser Thr Gly Thr Met Gly Gly Gly
   565 570 575
   Ser Ser Ala Gly Thr Pro Pro Lys Gln Gly Gly Ser Ala Pro Glu Gln
   580 585 590
<210> 9
   <211> 268
   <212> PRT
   <213> Mus musculus
<400> 9
   Met Glu Asp Phe Leu Leu Ser Asn Gly Tyr Gln Leu Gly Lys Thr Ile
   1 5 10 15
   Gly Glu Gly Thr Tyr Ser Lys Val Lys Glu Ala Phe Ser Lys Lys His
   20 25 30
   Gln Arg Lys Val Ala Ile Lys Ile Ile Asp Lys Met Gly Gly Pro Glu
   35 40 45
   Glu Phe Ile Gln Arg Phe Leu Pro Arg Glu Leu Gln Ile Val Arg Thr
   50 55 60
   Leu Asp His Lys Asn Ile Ile Gln Val Tyr Glu Met Leu Glu Ser Ala
   65 70 75 80
   Asp Gly Lys Ile Tyr Leu Val Met Glu Leu Ala Glu Gly Gly Asp Val
   85 90 95
   Phe Asp Cys Val Leu Asn Gly Gly Pro Leu Pro Glu Ser Arg Ala Lys
   100 105 110
   Ala Leu Phe Arg Gln Met Val Glu Ala Ile Arg Tyr Cys His Gly Cys
   115 120 125
   Gly Val Ala His Arg Asp Leu Lys Cys Glu Asn Ala Leu Leu Gln Gly
   130 135 140
   Phe Asn Leu Lys Leu Thr Asp Phe Gly Phe Ala Lys Val Leu Pro Lys
   145 150 155 160
   Ser Arg Arg Glu Leu Ser Gln Thr Phe Cys Gly Ser Thr Ala Tyr Ala
   165 170 175
   Ala Pro Glu Val Leu Gln Gly Ile Pro His Asp Ser Lys Lys Gly Asp
   180 185 190
   Val Trp Ser Met Gly Val Val Leu Tyr Val Met Leu Cys Ala Ser Leu
   195 200 205
   Pro Phe Asp Asp Thr Asp Ile Pro Lys Met Leu Trp Gln Gln Gln Lys
   210 215 220
   Gly Val Ser Phe Pro Thr His Leu Gly Ile Ser Thr Glu Cys Gln Asp
   225 230 235 240
   Leu Leu Lys Arg Leu Leu Glu Pro Asp Met Ile Leu Arg Pro Ser Ile
   245 250 255
   Glu Glu Val Ser Trp His Pro Trp Leu Ala Ser Thr
   260 265
<210> 10
   <211> 1020
   <212> DNA
   <213> Mus musculus
<400> 10
   gcacgagatg ttctagccct ggaggcagct gtgaatgaag tccttggggg gaaaagaagc 60
   aggccgaggg cgatggtgga gtagagctgc ctcgcagagg cagcatgagc tgagagggtg 120
   acaagaagga ggcgctacac agcatggagg actttctact ctccaatggg tatcagctgg 180
   gcaagaccat tggggaaggg acctactcaa aagtcaaaga agcattttcc aaaaaacatc 240
   aacgaaaagt ggcaattaaa attatagaca agatgggagg gccagaagag tttatccaga 300
   gattcctgcc tcgtgagctc cagattgtcc gtaccctgga ccacaaaaac atcatccagg 360
   tgtatgagat gctggagtca gcagatggaa aaatctacct ggtgatggag ctggctgagg 420
   gaggggatgt ctttgactgt gtgctgaacg gagggccact tcccgagagc cgggccaagg 480
   ccctcttccg ccagatggtt gaggctattc gctattgcca tggctgtggc gtggcccacc 540
   gggaccttaa gtgtgagaac gccttgttgc agggcttcaa cctgaagctg accgactttg 600
   gctttgccaa ggtgctaccc aagtcacgca gggagctgag ccagaccttc tgtggcagca 660
   cagcctatgc cgcccctgag gtgctacagg gcatacccca tgatagcaag aaaggtgatg 720
   tctggagcat gggtgtggtc ctgtatgtaa tgctctgtgc aagtctacct tttgacgaca 780
   cagatatccc caagatgctg tggcagcagc agaagggggt gtccttcccc actcatttgg 840
   gcatctcaac cgaatgccag gacctgctca agcggctcct ggaaccagac atgatactcc 900
   ggccttcaat cgaagaagtt agttggcacc catggctagc aagcacttga taaaagcaat 960
   ggcaagtcct ccccaataaa gtagggggag aaagcaaact gaaaaaaaaa aaaaaaaaaa 1020
<210> 11
   <211> 24
   <212> DNA
   <213> synthetic construct
<220>
   <221> primer_bind
   <222> (1)..(24)
<400> 11
   cgtggatcca waggaccasa crtc 24
<210> 12
   <211> 20
   <212> DNA
   <213> synthetic construct
<220>
   <221> primer_bind
   <222> (1)..(20)
<223> The n at position 15 represents any nucleotide selected from a, t, c, or g
<400> 12
   attcggatcc acmgngayyt 20
<210> 13
   <211> 24
   <212> DNA
   <213> synthetic construct
<220>
   <221> primer_bind
   <222> (1) .. (24)
<400> 13
   catcaccttt cttgctatca tggg 24
<210> 14
   <211> 21
   <212> DNA
   <213> synthetic construct
<220>
   <221> primer_bind
   <222> (1) .. (21)
<400> 14
   tgtgagaacg ccttgttgca g 21
<210> 15
   <211> 23
   <212> DNA
   <213> synthetic construct
<220>
   <221> primer_bind
   <222> (1) .. (23)
<400> 15
   gacatcacct tttttgctat cgt 23
<210> 16
   <211> 27
   <212> DNA
   <213> synthetic construct
<220>
   <221> primer_bind
   <222> (1)..(27)
<400> 16
   ccatcctaat acgactcact atagggc 27
<210> 17
   <211> 24
   <212> DNA
   <213> synthetic construct
<220>
   <221> primer bind
   <222> (1)..(24)
<400> 17
   gcaggtgaga atgttctaac gctg 24
<210> 18
   <211> 24
   <212> DNA
   <213> synthetic construct
<220>
   <221> primer_bind
   <222> (1)..(24)
<400> 18
   tctcccccta ctttattgga gagc 24

## Description

### US Government Rights

This invention was made with United States Government support under Grant Nos. HD 06274, HD 22732, HD 38082, and CA 06927, awarded by National Institutes of Health. The United States Government has certain rights in the invention.

### Claim to Priority

This application claims priority under 35 USC §199(e) to US Provisional Application Serial No. 60/246,939, filed November 9, 2000 and 60/264,921, filed January 30, 2001.

### Field of the Invention

The present invention is directed to a method for identifying antagonists of tssk kinase activity, the method comprising providing an *in vitro* kinase assay composition, said composition comprising a labeled source of phosphate, a tssk substrate comprising an amino acid sequence of SEQ ID NO: 8 and a tssk kinase, wherein the tssk kinase comprises SEQ ID NO: 6; measuring the rate of phosphorylation of the substrate in the presence and the absence of a candidate inhibitory compound; and identifying antagonist compounds that decrease tssk kinase activity to the kinase activity observed in the assays conducted in the absence of the antagonist compound.

### Background of the Invention

Spermatogenesis, the process in which functional sperm cells are produced in the testis, involves specific interaction between the developing germ cells and their supporting Sertoli cells as well as hormonal regulation by the androgen-producing Leydig cells. The general organization of spermatogenesis is essentially the same in all mammals and can be divided into three distinct phases: 1) The initial phase is the proliferative or spermatogonial phase during which spermatogonia undergo mitotic division and generate a pool of spermatocytes; 2) the meiotic phase, that yields the haploid spermatids; and 3) spermiogenesis whereby each round spermatid differentiates into a spermatozoon. Although the molecular mechanisms regulating the first two phases have been relatively well characterized, the molecular basis of spermiogenesis is largely unknown.

Mammalian spermiogenesis, the postmeiotic phase of spermatogenesis, is characterized by dramatic morphological changes that occur in the haploid spermatid. Some of these changes include the formation of the acrosome and its contents, the condensation and reorganization of the chromatin, the elongation and species-specific reshaping of the cell, and the assembly of the flagellum. These events result from changes in both gene transcription and protein translation that occurs during this developmental period. Some of the proteins translated in the haploid spermatid will remain in the morphologically mature sperm after it leaves the testis. Taking this into consideration, proteins that are synthesized during spermatogenesis might be necessary for spermatid differentiation and/or for sperm function during fertilization.

The present invention relates to signaling events in mammalian sperm that regulate the functions of this highly differentiated cell. More particularly the invention relates to signal transduction that modulates the acquisition of sperm fertilizing capacity. After ejaculation, sperm are able to move actively but lack fertilizing competence. They acquire the ability to fertilize in the female genital tract in a time-dependent process called capacitation. Capacitation has been demonstrated to be accompanied by the protein phosphorylation of several proteins on both serine/threonine and tyrosine residues, and that protein tyrosine phosphorylation is regulated downstream by a cAMP/PKA pathway that involves the crosstalk between these two signaling pathways. With the exception of PKA, the other kinase(s) involved in the regulation of capacitation are still unknown.

Additional protein kinases have been shown to be involved in spermatogenesis, however, only a few of them are exclusively expressed in germ cells or in the testis (Jinno et al, 1993, Cell Biol 13, 4146-56; Nayak et al, 1998, Mech Dev 74, 171-4; Shalom & Don, 1999, Mol Reprod Dev 52, 392-405; Toshima et al, 1998, Biochem Biophys Res Commun 249, 107-12; Toshima et al, 1999, J Biol Chem 274, 12171-6; Tseng et al, 1998, DNA Cell Biol 17, 823-33 ; Walden & Cowan, 1993, Mol Cell Biol 13, 7625-35). Examples of testis-specific kinases are the recently described mouse genes, tssk 1, 2 and 3 (Bielke et al., 1994, Gene 139, 235-9; Kueng et al, 1997, J Cell Biol 139, 1851-9; Zuercher et al., 2000, Mech Dev 93, 175-7). The function of the tssk kinase family is unknown. However, since the members of this family are expressed postmeiotically during spermiogenesis, it is hypothesized that they have a role in germ cell differentiation, or later on in sperm function. Therefore, it is anticipated that compounds that interfere with the function of this kinase family could be utilized as contraceptive agents.

Despite the availability of a range of contraceptive methods, over 50 % of pregnancies are unintended worldwide and in the United States. Thus, there is a critical need for contraception that better fits the diverse needs of women and men and takes into consideration different ethnic, cultural and religious values. Except for the use of condoms or vasectomy, the availability of contraceptive methods for men is very limited.

The importance of protein kinases in most physiological processes suggests that inhibition of tssk kinase family activity with a specific drug could inhibit fertilization. In accordance with one aspect of the present invention, the tssk kinase family is used as a target for the development of novel drugs. In particular, the sperm-specific tssk gene products will be used to screen for specific inhibitors of tssk kinase activity and these inhibitors will be used either alone or in conjunction with other contraceptive agents to prevent unintended pregnancies. Advantageously, the unique sequence of the members of the tssk kinase family supports the likelihood of finding specific inhibitors for their activity. Finally, if these kinases remain in the sperm after spermatogenesis and have a role in sperm physiology, design of specific tssk kinase inhibitors could be used both in male and female in order to prevent fertilization.

### Summary of the Invention

The present invention describes the human sperm-specific tssk kinase gene family and their corresponding proteins. More particularly, the present invention describes the human tssk1, tssk2 and tssk 3 kinases and the use of those kinases to identify agonists or antagonists of tssk kinase activity. The present invention also describes antibodies raised against the human tssk1, tssk2 and tssk 3 kinases and the use of these antibodies as diagnostic tools. The present invention is particularly directed to a method for identifying antagonists of tssk kinase activity, the method comprising providing an *in vitro* kinase assay composition, said composition comprising a labeled source of phosphate, a tssk substrate comprising an amino acid sequence of SEQ ID NO: 8 and a tssk kinase, wherein the tssk kinase comprises SEQ ID NO: 6; measuring the rate of phosphorylation of the substrate in the presence and the absence of a candidate inhibitory compound; and identifying antagonist compounds that decrease tssk kinase activity to the kinase activity observed in the assays conducted in the absence of the antagonist compound.

### Brief Description of the Drawings

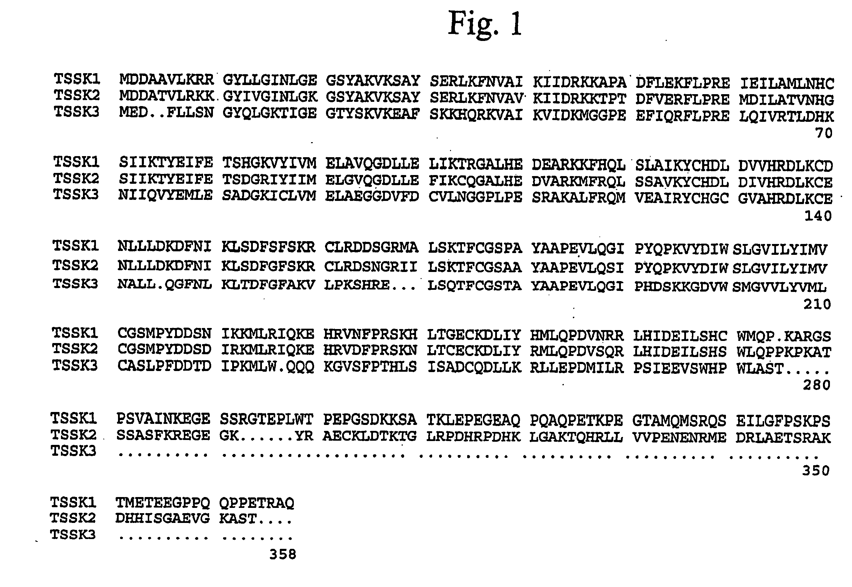

Fig. 1. Comparison between the amino acid sequences of human tssk 1, human tssk 2, and human tssk 3. As seen in Fig. 1 the amino acid sequences between the three proteins diverge near the carboxy terminus.

### Detailed Description of the Invention

### Definitions

In describing and claiming the invention, the following terminology will be used in accordance with the definitions set forth below.

As used herein, "nucleic acid," "DNA," and similar terms also include nucleic acid analogs, i.e. analogs having other than a phosphodiester backbone. For example, the so-called "peptide nucleic acids," which are known in the art and have peptide bonds instead of phosphodiester bonds in the backbone, are considered within the scope of the present invention.

The term "peptide" encompasses a sequence of 3 or more amino acids wherein the amino acids are naturally occurring or synthetic (non-naturally occurring) amino acids. Peptide mimetics include peptides having one or more of the following modifications:
1. peptides wherein one or more of the peptidyl --C(O)NR-- linkages (bonds) have been replaced by a non-peptidyl linkage such as a --CH₂-carbamate linkage (-CH₂OC(O)NR-), a phosphonate linkage, a -CH₂-sulfonamide (-CH ₂--S(O)₂NR--) linkage, a urea (--NHC(O)NH--) linkage, a -CH₂ -secondary amine linkage, or with an alkylated peptidyl linkage (--C(O)NR--) wherein R is C₁₋C₄ alkyl;
2. peptides wherein the N-terminus is derivatized to a --NRR₁ group, to a -- NRC(O)R group, to a --NRC(O)OR group, to a --NRS(O)₂R group, to a -NHC(O)NHR group where R and R₁ are hydrogen or C₁-C₄ alkyl with the proviso that R and R₁ are not both hydrogen;
3. peptides wherein the C terminus is derivatized to --C(O)R₂ where R₂ is selected from the group consisting of C₁-C₄ alkoxy, and -NR₃R₄ where R₃ and R₄ are independently selected from the group consisting of hydrogen and C₁-C₄ alkyl.

Naturally occurring amino acid residues in peptides are abbreviated as recommended by the IUPAC-IUB Biochemical Nomenclature Commission as follows: Phenylalanine is Phe or F; Leucine is Leu or L; Isoleucine is Ile or I; Methionine is Met or M; Norleucine is Nle; Valine is Vat or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; Glycine is Gly or G, and X is any amino acid. Other naturally occurring amino acids include, by way of example, 4-hydroxyproline, 5-hydroxylysine, and the like.

Synthetic or non-naturally occurring amino acids refer to amino acids which do not naturally occur *in vivo* but which, nevertheless, can be incorporated into the peptide structures described herein. The resulting "synthetic peptide" contain amino acids other than the 20 naturally occurring, genetically encoded amino acids at one, two, or more positions of the peptides. For instance, naphthylalanine can be substituted for trytophan to facilitate synthesis. Other synthetic amino acids that can be substituted into peptides include L-hydroxypiopyl, L-3,4-dihydroxyphenylalanyl, alpha-amino acids such as L-alpha-hydroxylysyl and D-alpha-methylalanyl, L-alpha.-methylalanyl, beta.-amino acids, and isoquinolyl. D amino acids and non-naturally occurring synthetic amino acids can also be incorporated into the peptides. Other derivatives include replacement of the naturally occurring side chains of the 20 genetically encoded amino acids (or any L or D amino acid) with other side chains.

As used herein, the term "conservative amino acid substitution" are defined herein as exchanges within one of the following five groups:
I. Small aliphatic, nonpolar or slightly polar residues:
   Ala, Ser, Thr, Pro, Gly;
II. Polar, negatively charged residues and their amides:
   Asp, Asn, Glu, Gln;
III. Polar, positively charged residues:
   His, Arg, Lys;
IV. Large, aliphatic, nonpolar residues:
   Met Leu, Ile, Val, Cys
V. Large, aromatic residues:
   Phe, Tyr, Trp

As used herein, the term "purified" and like terms relate to the isolation of a molecule or compound in a form that is substantially free of contaminants normally associated with the molecule or compound in a native or natural environment.

As used herein, the term "biologically active fragments" or "bioactive fragment" of an tssk polypeptide encompasses natural or synthetic portions of the full-length protein that are capable of specific binding to their natural ligand.

"Operably linked" refers to a juxtaposition wherein the components are configured so as to perform their usual function. For example, control sequences or promoters operably linked to a coding sequence are capable of effecting the expression of the coding sequence.

The term "non-native promoter" as used herein refers to any promoter that has been operably linked to a coding sequence wherein the coding sequence and the promoter are not naturally associated (i.e. a recombinant promoter/coding sequence construct).

As used herein, a transgenic cell is any cell that comprises a nucleic acid sequence that has been introduced into the cell in a manner that allows expression of the a gene encoded by the introduced nucleic acid sequence.

### The Invention

The present disclosure describes a family of kinases (the tssk kinase family) that are expressed exclusively in germ cells of humans and mice. The mouse homologs of the human tssk genes were previously described and were found to be expressed postmeiotically in male germ cells. (Bielke et al., 1994, *Gene* **139**, 235-9; Kueng et al., 1997, *J Cell Biol* **139**, 1851-9) Using a combination of two yeast hybrid technology and coimmunoprecipitation, Kueng et al. (1997) found that mouse tssk 1 and 2 bind and phosphorylate a protein of 54 Kda, that represents the tssk substrate. This tssk substrate has been designated tsks. The tsks protein is also testis-specific and its developmental expression suggests that it is postmeiotically expressed in germ cell. The mouse cDNA sequence of the tssk substrate was previously reported (Kueng et al., 1997) and was used to search the EST data base. A human EST homologue AL041339 was found and used to generate sense and antisense primers for obtaining the full length clone by 5' and 3' RACE using human testis marathon ready cDNA (Clontech, Inc.). The full length nucleotide sequence of human tsks is described as SEQ ID NO: 7 and the deduced protein sequences is provided as SEQ ID NO: 8.

The developmental expression pattern of the tssk kinases, as well as the general relevance of kinases in physiological processes led applicants to believe that this family of testis-specific kinases has a role in spermatogenesis. The finding that the tssk kinase family and one of the putative substrates are expressed at the same time during spermatogenesis is relevant to the potential use of these proteins as contraceptive targets. Accordingly, one aspect describes the isolation of the human tssk homologs and their use in isolating contraceptive agents.

Since sperm are transcriptionally and translationally inactive, cloning and characterizing candidate sperm protein kinases at the molecular level, requires the use of RNA isolated from male germ cells. RNA transcripts expressed in the male germ cell lineage might ultimately be important in sperm function; however, it is also possible that such transcripts function during testicular spermatogenesis. Using this methodology a unique cDNA was cloned from mouse male germ cells that encodes a putative protein kinase of the ser/thr protein kinase subfamily. That kinase (tssk3b) is specifically expressed postmeiotically in murine male germ cells. In accordance with the present description a unique testis-specific mouse tssk gene is disclosed. The nucleic acid sequence and amino acid sequence of tssk3b is disclosed as SEQ ID NO 10 and SEQ ID NO:9, respectively.

The human homologue of tssk3b has also been cloned (and designated human tssk3) and exhibits 98 % homology to the putative mouse protein at the protein level. The nucleotide sequence and amino acid sequence of human tssk3 is disclosed as SEQ ID NO 3 and SEQ ID NO: 6, respectively. Recently, a similar mouse protein kinase was described and identified as a testis-specific serine kinase 3 (mouse tssk3) (Zuercher et al., 2000, Mech Dev 93, 175-7), a member of a small family of testis-specific protein kinases (Bielke et al., 1994, Gene 139, 235-9; Kueng et al., 1997, J Cell Biol 139, 1851-9). Despite this homology, both the human tssk3 and the mouse tssk3b cDNAs cloned and described in the present application demonstrate differences with mouse tssk3 in several amino acids.

Using the predicted mouse tssk 1 and 2 amino acid sequences (as described by Kueng et al., 1997) for the purposes of searching the gene bank, a genome sequence of both mouse tssk 1 and tssk2 was obtained. The 3' end and 5' end of the coding region of these genomic mouse genes were then used to design sense and antisense primers, respectively, and used to isolate the human homologs using PCR technology (see Example 2). The amplified cDNA fragments were cloned using the TOPO TA cloning kit (Invitrogen) and sequenced. An amplified human tssk1 gene was approximately 1.3 kb in size and the isolated human tssk 2 gene was approximately 1.2 kb in size. The nucleic acid sequence and amino acid sequence of human tssk1 is disclosed as SEQ ID NO: 1 and SEQ ID NO: 4, respectively. The nucleic acid sequence and amino acid sequence of human tssk2 is disclosed as SEQ ID NO: 2 and SEQ ID NO: 5, respectively.

In accordance with the present description a purified polypeptide is disclosed comprising the amino acid sequence of SEQ ID NO : 4, SEQ ID NO: 5, SEQ ID NO: 6 or SEQ ID NO: 9, or an amino acid sequence that differs from SEQ ID NO: 1 or SEQ ID NO: 3 by one or more conservative amino acid substitutions. More preferably the purified polypeptide comprises an amino acid sequence that differs from SEQ ID NO: 1 or SEQ ID NO: 3 by less than 5 conservative amino acid substitutions, and more preferably by 2 or less conservative amino acid substitutions.

The purified polypeptide may comprise the amino acid sequence of SEQ ID NO: 4, SEQ ID NO: 5 or SEQ ID NO: 6. These polypeptides may include additional amino acid sequences to assist in the purification of recombinantly produced polypeptides. The purified polypeptide may comprise an amino acid sequence selected from the group consisting of SEQ ID NO: 4, SEQ ID NO: 5 and SEQ ID NO: 6 and a peptide tag, wherein the peptide tag is linked to the tssk peptide sequence. Suitable expression vectors for expressing such fusion proteins and suitable peptide tags are known to those skilled in the art and commercially available. In one embodiment the tag comprises a His tag (see Example 4). In one embodiment the purified polypeptide used herein comprises the amino acid sequence of SEQ ID NO: 8 or the amino acid sequence of SEQ ID NO: 8 linked to a peptide tag.

The present invention further describes a purified polypeptide that comprises a portion of a tssk polypeptide. More particularly the tssk polypeptide portion consists of natural or synthetic portions of a full-length polypeptide selected from the group consisting of SEQ ID NO: 4, SEQ ID NO: 5, SEQ ID NO: 6 and SEQ ID NO: 9 that are capable of specific binding to their natural ligand. The human tssk fragment retains its ability to bind to tsks.

The present invention also describes nucleic acid sequences that encode human tssk. E.g., a nucleic acid sequence is described comprising the sequence of SEQ ID NO: 1, SEQ ID NO: 2, SEQ ID NO: 3, SEQ ID NO: 10 or fragments thereof. Furthermore a purified nucleic acid sequence is described, selected from the group consisting of SEQ ID NO: 1, SEQ ID NO: 2 and SEQ ID NO: 3.

The present disclosure also describes nucleic acids that hybridize under stringent or highly stringent conditions (as defined herein) to all or a portion of the nucleotide sequence represented by SEQ ID NO: 1, SEQ ID NO: 2, SEQ ID NO: 3 or SEQ ID NO: 10, or their complements. The hybridizing portion of the hybridizing nucleic acids is typically at least 15 (e.g., 20, 25, 30, or 50) nucleotides in length. Hybridizing nucleic acids of the type described herein can be used, for example, as a cloning probe, a primer (e.g., a PCR primer), or a diagnostic probe to detect the expression of the human tssk gene.

Nucleic acid duplex or hybrid stability is expressed as the melting temperature or Tm, which is the temperature at which a nucleic acid duplex dissociates into its component single stranded DNAs. This melting temperature is used to define the required stringency conditions. Typically a 1% mismatch results in a 1°C decrease in the·Tm, and the temperature of the final wash in the hybridization reaction is reduced accordingly (for example, if two sequences having > 95% identity, the final wash temperature is decreased from the Tm by 5°C). In practice, the change in Tm can be between 0.5°C and 1.5°C per 1% mismatch.

The present description also discloses the nucleic acid sequence of SEQ ID NO: 1, SEQ ID NO: 2 or SEQ ID NO: 3 and nucleic acid sequences that hybridize to those sequences (or their complement) under stringent or highly stringent conditions. In accordance with the present disclosure highly stringent conditions are defined as conducting the hybridization and wash conditions at no lower than -5°C Tm. Stringent conditions are defined as involve hybridizing at 68°C in 5x SSC/5x Denhardt's solution/1.0% SDS, and washing in 0.2x SSC/0.1% SDS at 68°C. Moderately stringent conditions include hybridizing at 68°C in 5x SSC/5x Denhardt's solution/1.0% SDS and washing in 3x SSC/0.1% SDS at 42°C. Additional guidance regarding such conditions is readily available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The present disclosure is also describes recombinant human tssk gene constructs. The recombinant gene construct may comprise a non-native promoter operably linked to a nucleic acid sequence selected from the group consisting of SEQ ID NO: 1, SEQ ID NO: 2, SEQ ID NO: 3, SEQ ID NO: 7 and SEQ ID NO: 10. The recombinant gene construct comprises a non-native promoter operably linked to a nucleic acid sequence selected from the group consisting of SEQ ID NO: 1, SEQ ID NO: 2 and SEQ ID NO: 3. The non-native promoter is preferably a strong constitutive promoter that allows for expression in a predetermined host cell. These recombinant gene constructs can be introduced into host cells to produce transgenic cell lines that synthesize the tssk gene products. Host cells can be selected from a wide variety of eukaryotic and prokaryotic organisms, and two preferred host cells are *E. coli* and yeast cells.

In accordance with the disclosure, a nucleic acid sequence selected from the group consisting of SEQ ID NO: 1, SEQ ID NO: 2, SEQ ID NO: 3 and SEQ ID NO: 10 are inserted into a eukaryotic or prokaryotic expression vector in a manner that operably links the gene sequences to the appropriate regulatory sequences, and human tssk is expressed in the appropriate eukaryotic or prokaryotic cells host cell. Suitable eukaryotic host cells and vectors are known to those skilled in the art. The baculovirus system is also suitable for producing transgenic cells and synthesizing the tssk genes of the present disclosure. One aspect herein is directed to transgenic cell lines that contain recombinant genes that express human tssk and fragments of the human tssk coding sequence. As used herein a transgenic cell is any cell that comprises an exogenously introduced nucleic acid sequence. More preferably the introduced nucleic acid is sufficiently stable in the transgenic cell (i.e. incorporated into the cell's genome, or present in a high copy plasmid) to be passed on to progeny cells. The transgenic cell may be a human cell and comprise a nucleic acid sequence selected from the group consisting of SEO ID NO: 1, SEQ ID NO: 2, SEQ ID NO: 3, SEQ ID NO: 7 and SEQ ID NO: 10. Preferably the nucleic acid sequence is selected from the group consisting of SEQ ID NO: 1, SEQ ID NO: 2 and SEQ ID NO: 3. The present disclosure also describes non-human transgenic organisms wherein one or more of the cells of the transgenic organism comprise a recombinant gene that expresses the human tssk.

The present disclosure also disclosure a method for producing human tssk. The method comprises the steps of introducing a nucleic acid sequence comprising sequences encoding the human tssk into a host cell, and culturing the host cell under conditions that allow for expression of the introduced human tssk gene. The promoter may be a conditional or inducible promoter, alternatively the promoter may be a tissue specific or temporal restricted promoter (i.e. operably linked genes are only expressed in a specific tissue or at a specific time). The synthesized tssk can be purified using standard techniques and used in high throughput sceeens to identify inhibitors of tssk activity. Alternatively, the recombinantly produced tssk polypeptides, or fragments thereof are used to generate antibodies against the tssk polypeptides. The recombinanatly produced tssks are also used to obtain crystal structures. Such structures would allow for crystallography analysis that would lead to the design of specific drugs to inhibit tssk function.

Preferably, the nucleic acid sequences encoding the sperm-specific kinase are inserted into a suitable expression vector in a manner that operably links the gene sequences to the appropriate regulatory sequences for expression in the preselected host cell. Suitable host cells, vectors and methods of introducing the DNA constructs into cells are known to those skilled in the art. In particular, nucleic acid sequences encoding the sperm-specific kinase may be added to a cell or cells *in vitro* or *in vivo* using delivery mechanisms such as liposomes, viral based vectors, or microinjection.

The present disclosure also describes a composition comprising a peptide having the sequence selected from the group consisting of SEQ ID NO: 4, SEQ ID NO: 5 and SEQ ID NO: 6 or an antigenic fragment thereof. The antigenic fragment may consist of the sequence of SEQ ID NO: 4 or SEQ ID NO: 6. The compositions can be combined with a pharmaceutically acceptable carrier or adjuvants and administered to a mammalian species to induce an immune response.

The present disclosure also describes isolated antibodies that are generated against human tssk or fragments thereof. Antibodies to human tssk may be generated using methods that are well known in the art. In accordance therewith an antibody is described that specifically binds to a polypeptide selected from the group consisting of SEQ ID NO: 4, SEQ ID NO: 5, SEQ ID NO: 6, SEQ ID NO: 8 and SEQ ID NO: 9. Furthermore, antibodies are described that bind to a polypeptide selected from the group consisting of SEQ ID NO: 4, SEQ ID NO: 5 and SEQ ID NO: 6. The antibody may be a monoclonal antibody. The antibodies may be used with or without modification, and may be labeled by joining them, either covalently or non-covalently, with a reporter molecule. In addition, the antibodies can be formulated with standard carriers and optionally labeled to prepare therapeutic or diagnostic compositions.

The present disclosure also describes a method for detecting the presence of human tssk. The method comprises the steps of contacting a sample with a labeled antibody that specifically binds to human tssk, removing unbound and non-specific bond material and detecting the presence of the labeled antibody. The labeled compound may comprise an antibody that is labeled directly or indirectly (i.e. via a labeled secondary antibody). In particular, the tssk antibodies described herein can be used to confirm the expression of tssk as well as its cellular location, or in assays to monitor patients being treated with human soluble tssk or inhibitors.

In accordance with one aspect of the present invention, the tssk kinase family is used as a target for the development of novel drugs. Progress in the field of small molecule library generation using combinatorial chemistry methods coupled to high-throughput screening has accelerated the search for ideal cell-permeable inhibitors. In addition, structural-based design using crystallographic methods has improved the ability to characterize in detail ligand-protein interaction sites that can be exploited for ligand design.

In one embodiment, the present invention provides methods of screening for agents, small molecules, or proteins that interact with polypeptides comprising the sequence of tssk1, tssk2, tssk3 or bioactive fragments thereof. As used herein, the term "biologically active fragments" or "bioactive fragment" of tssk1, tssk2, tssk3 encompasses natural or synthetic portions of the native peptides that are capable of specific binding to at least one of the natural ligands of the respective native tssk1, tssk2, tssk3 polypeptide, including tsks. The invention encompasses both *in vivo* and *in vitro* assays to screen small molecules, compounds, recombinant proteins, peptides, nucleic acids, antibodies *etc.* which bind to or modulate the activity of tssk1, tssk2, tssk3 and are thus useful as therapeutics or diagnostic markers for fertility.

In one embodiment of the present invention tssk polypeptides, selected from SEQ ID NO: 6, are used to isolate ligands that bind to tssk under physiological conditions. The method comprises the steps of contacting the tssk polypeptides with a mixture of compounds under physiological conditions, removing unbound and non-specifically bound material, and isolating the compounds that remain bound to the tssk polypeptides. Typically, the tssk polypeptides will be bound to a solid support, using standard techniques, to allow for rapid screening of compounds. The solid support can be selected from any surface that has been used to immobilize biological compounds and includes but is not limited to polystyrene, agarose, silica or nitrocellulose. In one embodiment the solid surface comprises functionalized silica or agarose beads. Screening for such compounds can be accomplished using libraries of pharmaceutical agents and standard techniques known to the skilled practitioner.

Ligands that bind to the tssk polypeptides can then be further analyzed for agonists and antagonists activity through the use of an *in vitro* kinase assay as described in Example 7. Inhibitors of tssk kinase activity have potential use as agents that prevent maturation/capacitation of sperm. Such inhibitors can be formulated as pharmaceutical compositions and administered to a subject to block spermatogenesis and provide a means for contraception.

In accordance with one embodiment, specific inhibitors of the human tssk kinase activity are identified through the use of an *in vitro* kinase assay that is capable to detecting phosphorylation events. In one embodiment the method of identifying inhibitors of tssk kinase activity comprises combining a labeled source of phosphate with one or more of the human tssk polypeptides in the presence of one or more potential inhibitory compounds. As described in Example 4 the tssk proteins have the property to become autophosphorylated. Therefore by comparing the rate of autophosphorylation that occurs in the presence and absence of the candidate inhibitory compound, specific inhibitory compounds can be identified. In one preferred embodiment a tssk substrate is provided and the assay is based on measuring the rate of phosphorylation of said substrate in the presence and absence of the candidate inhibitory compounds. Preferably large numbers of compounds will screened using high through put techniques to identify tssk specific inhibitory compounds.

In accordance with the present invention specific inhibitors of the tssk kinase activity are identified by providing an *in vitro* kinase assay composition, wherein the composition comprises a labeled source of phosphate, a tssk substrate and a tssk kinase selected from SEQ ID NO: 6. The rate of tssk substrate phosphorylation will be determined under controlled conditions in the absence of any inhibitory compounds, and then the identical conditions will be used to measure the rate of phosphorylation of the tssk substrate when the assay is run in the presence of one or more potential inhibitory compounds. Those compounds that decrease the activity of the tssk kinase will be identified and tested to determine if the inhibitory effect is specific to the tssk kinase.

In one embodiment the method for identifying human tssk inhibitors comprises the steps of providing a labeled source of phosphate, a tssk substrate and a tssk kinase selected from SEQ ID NO: 6, contacting that composition with one or more potential inhibitory compounds and measuring the rate of phosphorylation. The labeled source of phosphate is preferably [γ32P] ATP and the tssk substrate comprises an amino acid sequence of SEQ ID NO: 8. The kinase assay can also be conducted with two or more of the human tssk kinases present to confirm the activity of the inhibitor to all three kinases, e.g. selected from the group consisting of SEQ ID NO:4, SEQ ID NO:5, SEQ ID NO:6 and SEQ ID NO:9.

### Example 1

### Isolation of a Novel Mouse Testis Specific Serine/Threonine Kinase

Reverse transcription-polymerase chain reaction (RT- PCR) using degenerate oligonucleotides corresponding to conserved regions present in protein kinases resulted in the isolation of a novel member of the testis-specific serine/threonine kinase. This PCR fragment recognized a 1020 bp transcript in male germ cells by northern blot analysis. Using this fragment as a probe, a full length cDNA was cloned from a mouse mixed germ cell cDNA library. This cDNA has an open reading frame of 804 bases encoding a protein of 268 amino acids. Tissue expression analysis revealed that this protein kinase is developmentally expressed in mouse testicular germ cells and is not present in brain, ovary, kidney, liver or early embryonic cells.

This novel putative serine/threonine protein kinase (SEQ ID NO: 9) is almost identical to tssk3 (Zuercher et al., 2000, Mech Dev 93, 175-7), a recently described mouse testis-specific protein kinase, with the exception of several base pair deletions that result in a shift in the coding region and an alteration of 22 amino acids (residues 109 to 131). The human homologue of this novel protein kinase (SEQ ID NO: 6) was subsequently cloned and displayed expression exclusively in the testis. Fluorescence *in situ* hybridization (FISH) using both the human and mouse cDNA clones revealed syntenic localization on chromosomes 1p34-35 and 4E, respectively. Due to the homology with tssk3 this novel protein kinase is named tssk3b.

### Materials and Methods

### Isolation of Spermatogenic Cell Populations

Purified populations of pachytene spermatocytes, round spermatids, and condensing spermatids were prepared from decapsulated testes of adult mice (CD-1; Charles Rivers) by sequential dissociation with collagenase and trypsin-DNase 1 (Bellve et al., 1977; Romrell et al., 1976). The cells were separated into discrete populations by sedimentation velocity at unit gravity in 2-4 % BSA gradients in enriched Krebs Ringer Bicarbonate Medium (EKRB) (Bellve et al., 1977; Romrell et al., 1976). The pachytene spermatocyte and round spermatid populations were each at least 85 % pure, while the condensing spermatid population was ~40-50 % pure (contaminated primarily with anucleated residual bodies and some round spermatids).

### RNA Isolation and Northern Blot Analysis

Total RNA from somatic tissues, testis of mice of defined ages, and isolated spermatogenic cells of adult mice was isolated by homogenizing the cells in 5 M guanidium isothiocyanate, 25 mM sodium citrate, pH 7.2, 0.5 % Sarkosyl, and 0.1 M 2-mercaptoethanol (Chirgwin et al., 1979). Lysates were centrifuged over cushions of 5.7 M CsCl, 0.1 M EDTA at 114,000 x g at 20°C overnight. Pellets were resuspended and extracted with phenol:chloroform, and the RNA was precipitated with ethanol. The integrity of the RNA was verified by ethidium bromide staining of the ribosomal RNA on 1% agarose gels. Equivalent amounts of RNA were subjected to electrophoresis in 1.2 % agarose gels containing formaldehyde (Sambrook et al., 1989). RNA was transferred to nitrocellulose paper, baked at 80°C for 2 hr, and prehybridized in 50% formamide, 5X Denhartdt's, 0.1% SDS, 100 µg/ml Torula RNA, 5X SSPE for a minimum of 1 hr at 42°C. The appropriate DNA probe was generated by PCR, radiolabeled with 32p-dCTP by the random-primed method and incubated with the blots (1 x 10⁶ cpm/ml) in 50% formamide, 5X Denhartdt's, 0.1% SDS, 100 µg/ml Torula RNA, 5X SSPE with 10 % dextran sulfate and hybridized at 42°C overnight Blots were washed in 2X SSPE, 0.1 % SDS and then in 0.1XSSPE, 0.1% SDS, both for 2x10 min at room temperature. After washing, the filters were air-dried and exposed to film at - 70°C with intensifying screens. Northern blots of human tissues were acquired from Clontech and hybridization was performed as described above.

### Reverse Transcription-Polymerase Chain Reaction

Reverse transcription-polymerase chain reaction (RT -PCR) was performed using Superscript II from Gibco-BRL according to the manufacturer's instructions, followed by the use of Taq Polymerase from Promega. The PCR conditions were as follows: 1 cycle of 2 min. at 95°C; 35 cycles of 1 min. at 95°C, 1 min at 55°C and 1 min at 72°C; the cycles were finished with 1 cycle of 7 min at 72°C and then chilled to 4°C. The DNA products were then analyzed on 2 % agarose gels. The following degenerate primers were used to target conserved regions of tyrosine kinases: for the reverse transcription step, a degenerate antisense 20-mer corresponding to the coding subregion IX common to the subfamily of tyrosine kinases with the addition of a 5' Eco RI consensus site for subcloning after three random nucleotides (CGTGGATCCA(A/T)AGGACCA(C/G)AC(A/G)TC; SEQ ID NO: 11); (2) For the PCR step the same primer was used together with a degenerate sense 20-mer corresponding to the subregion VIb common to the subfamily of tyrosine kinases, also a consensus site for Eco RI was introduced after four random nucleotides ATICGGATCCAC(A/C)G(A/C/T/G)GA(C/T)(C/T)T; SEQ ID NO: 12. After the PCR reaction was completed and the products analyzed, the cDNA was subcloned into a TOPO TA cloning vector according to the manufacturers instructions (In Vitrogen), the Eco RI subcloning consensus sites were not used. Minipreps from 35 positive colonies were prepared using a Qiagen kit and then sequenced using a T7 primer.

### Cloning of mouse tssk 3b

To clone tssk 3b, the unique sequence obtained by RT -PCR was used to design specific primers against this novel kinase. Primers A2 (antisense: CATCACCTTTCTTGCTATCATGGG; SEQ ID NO: 13) and S2 (sense:(TGTGAGAACGCCTTGTTGCAG; SEQ ID NO: 14) were used to obtained a PCR fragment of 169 bases. This PCR fragment was subsequently radiolabeled with 32p-dCTP by the random-primed method and used as a probe to screen a oligo-dT primed mixed germ cell cDNA library as previously described.

### Cloning of the human tssk3b homologue

Using the predicted mouse tssk3b amino acid sequence for the purposes of searching the human EST database, entry AI 553938 was pulled out in a BLAST search. This ESTsequence was used to design an antisense primer A3 (GACATCACCTTTTTTGCTATCGT; SEQ ID NO: 15). This primer and an adaptor primer (CCATCCTAATACGACTCACTATAGGGC; SEQ ID NO: 16) were used in 5' RACE using human testis marathon ready cDNA as the template (Clontech, Inc ). The PCR reaction mix was first heated for 10 min at 94°C and the mix then subjected to 40 cycles of PCR under the following condition: 94°C 30 sec, 55°C 30 sec, 72°C 2min. Following these cycles, the mix was incubated at 72°C for 10 min. AmpTaq Gold (Perkin Elmer) was used in place of conventional Taq polymerase for the PCR. The amplified cDNA fragment was cloned and sequenced using the TOPO TA cloning kit (Invitrogen). The 5' end of the cDNA sequence obtained following 5' RACE was used to design a sense primer S3 (GCAGGTGAGAATGTTCTAACGCTG; SEQ ID NO: 17); an antisense primer A4 (TCTCCCCCTACTTTATTGGAGAGC; SEQ ID NO: 18) was based on the 3' end of AI 553938. These two primers were then used to amplify the full length human tssk3b homologue from the same library using the conditions described above. The amplified 1.058 kb fragment was excised, cloned and sequenced using the University of Virginia sequencing facility. A C-termina1400 bp fragment of the cDNA library amplified tssk3b human homologue was used for northern blots of human tissues.

### DNA Sequencing and Computer Analysis

All sequencing was performed with the AmpliTaq, FS dye terminator cycle sequencing kit chemistry and the appropriate primers using a 373A DNA sequencer (PE Applied Biosystems, Foster City, CA). Ambiguities were resolved by sequencing the opposite strand. DNA and protein sequence analyses were performed using the MacVector TM (Kodak Scientific Imaging Systems, New Haven, CT) and SequencherTM (Gene Codes Corp., Ann Arbor, MI) software programs.

### Fluorescence In Situ Hybridization and Chromosomal Mapping

Fluorescence *in situ* hybridization (FISH) and detection of immunofluorescence were carried out as previously described (Bell et al., 1995, Cytogenet. Cell Genet, 70: 263-267). The 1 kb, mouse cDNA clone, as well as the corresponding 1 kb human homologue, were biotinylated by nick translation in a reaction containing 1 µg DNA, 20 µM each of dA TP , dCTP and dGTP (Perkin Elmer), 1 µM dTTP (Perkin Elmer), 25 mM Tris-HCl, pH 7.5,5 mM MgC12 (Sigma), 10 mM B-mercaptoethanol (Sigma), 10 µM biotin-16-dUTP (Boehringer Mannheim), 2 units DNA polymerase I/DNase I (GIBCO, BRL), and H₂O to a total volume of 50 µl. Probes were denatured and hybridized to metaphase spreads from human peripheral lymphocytes and mouse embryonic fibroblasts, respectively. The hybridized probe was detected with fluorescein-labeled avidin and the signal amplified by the addition of anti-avidin antibody (Oncor) and a second layer of fluorescein-labeled avidin. The chromosome preparations were counterstained with DAPI and observed with a Zeiss Axiophot epiflourescence microscope equipped with a cooled CCD camera (Photometrics, Tucson, AZ) operated by a Macintosh computer workstation. Digitized images of DAPI staining and FITC signals were captured, pseudocolored, and merged using Oncor version 1.6 software.

### Results

### Cloning of protein kinases from murine male germ cells

Mammalian sperm capacitation is accompanied by an increase in protein tyrosine phosphorylation of several proteins. Since mature sperm are not able to synthesize proteins and the presence of RNA in these cells is controversial, in order to identify protein kinases by RT-PCR for the purposes of ultimately deducing their function, RNA was first isolated from a mouse mixed germ cell population. Degenerate primers targeting conserved regions in the subfamily of tyrosine kinases were employed and, following TA cloning, 27 sequences obtained as described in Methods were analyzed and compared with sequences in Gene Bank. Although most of the sequences match known members of the tyrosine kinase subfamily, some of the sequences obtained matched members of the subfamily of ser/thr protein kinases.

Between known members of this subfamily such as B-Rafkinase, a sequence with homology to a novel family of ser/thr protein kinases participating in spermiogenesis was detected (Kueng et al., 1997, J Cell Biol 139, 1851-9)*.* To clone this novel ser/thr kinase, a 169 bases specific PCR fragment corresponding to this sequence was radiolabeled and a mouse mixed germ cell cDNA library was screened as described in Methods. A clone containing a 1.02 kb cDNA insert was obtained (SEQ ID NO: 10) and designated tssk-3b following the nomenclature of Kueng et al. Sequence analysis revealed a single open reading frame of 804 nucleotides encoding a 266 amino acids putative ser/thr protein kinase. The nucleotides flanking the start methionine conform well to Kozak consensus sequence. The 3 '-untranslated region displays a polyadenylation signal 21 nucleotides upstream of the poly(A) tail. The sequence contains all the expected conserved domains corresponding to a ser/thr kinase.

### Expression pattern of murine tssk3

The expression pattern of murine tssk-3b was investigated by northern blot analysis of total RNA from different mouse tissues using a tssk-3b specific probe corresponding to the full length transcript of tssk 3b. This probe recognized a single transcript of approximately 1 kb exclusively in the mixed mouse germ cell population prepared as described in Methods. At high exposures it is also possible to distinguish a 1.35 kb transcript also exclusively in germ cells, this transcript was not observed when the mouse germ cell library was screened and could represent a splicing alternative of tssk 3b. To further analyze the expression pattern of tssk-3b in the testis, a northern blot with RNA obtained from purified germ cells of the adult testis was probed. tssk-3b mRNA was found to be expressed postmeiotically in round and condensing spermatids but not in the meiotic pachytene spermatocytes. Mouse testes differentiate at d 11-12 of embryonic development and are populated by primordial germ cells. The first spermatogenic wave is initiated a few days after birth and spermatogonia differentiate to early spermatids before puberty. The differentiation to mature sperm is testosterone dependent and occurs after puberty.

To further investigate whether tssk-3b is expressed postmeiotically, total testis RNA was prepared from mice of different postnatal ages (1,3, 7, 10, 15, 20, 24, 30 and adult) and analyzed by northern blot analysis. Transcription of tssk-3b began between 20 and 24 days after birth confirming a postmeiotic expression of this mRNA.

These results demonstrate that the expression pattern of tssk 3b is similar to that of mouse tssk 1 and tssk 2 (Kueng et al., 1997, J Cell Biol 139, 1851-9), suggesting that tssk 3 mRNA expression is developmentally regulated and that its expression is stimulated postmeiotically around the onset of spermiogenesis.

To further analyze the expression pattern of tssk 3b, we have performed RT-PCR using specific primers in oocytes, metaphase II -arrested eggs and different stages of preimplantation embryo development. No PCR product was observed at any of these stages under conditions in which the correct sized tssk 3b PCR product could be amplified from mixed germ cell total RNA.

### Cloning and expression of the human homologue of tssk 3b

Using the mouse tssk 3 sequence, a human EST from germ cell tumor was identified by a BLAST search of the databases, and used in conjunction with 3' and 5' RACE to clone the full length human homologue (SEQ ID NO: 3) from an adapted ligated human testis cDNA library (Clontech) as described in Methods.

To investigate the expression pattern of tssk 3 in human tissues, a tissue northern blot (Clontech) was probed with a C terminal 400 bp fragment of the human tssk3 cDNA. 1 kb and 1.35 kb RNA transcripts were expressed exclusively in the testis. Similar to the mouse case, the 1.35 kb fragment could represent an alternative spliced transcript. Both the mouse tssk 3b and the human homologue tssk 3 of these novel ser/thr kinases have the highest homology (98 %) between each other, followed by mouse tssk3 (92 %) mouse tssk1 and mouse tssk 2 (56 %) suggesting that this kinase belongs to the same subfamily of novel ser/thr kinases. As mentioned, tssk 3 and tssk 3b have a very high homology (92%), the difference between these two sequences is restricted to a stretch of 22 aminoacids (residues 109 to 131 ). When this stretch is analyzed at the nucleotide level, three base pair deletions were observed in the mouse tssk 3 sequence that resulted in a shift in the coding region and an alteration of the aforementioned 22 amino acids. It is unclear at this moment the origin of these frame shifts, one possibility is that two different tssk 3 are present in mouse testis. More likely, one of these two sequences could have a small mistake in the sequence. Since the tssk 3 b human homologue was obtained independently, from the mouse cDNA clone and has 100 % homology at the amino acid level with its mouse homologue, applicants are confident about the accuracy of both the mouse 3b and the human tssk 3 sequences.

### Chromosomal mapping of human and murine tssk3.

The chromosomal location of tssk 3b has also been mapped by fluorescence in situ hybridization (FISH) using the full length human cDNA probe. Fluorescent signals were detected on chromosome 1 in all 20 metaphase spreads scored. Among a total of 109 signals observed, 49 ( 45% ) were on 1 p. All chromosome-specific signals were localized to 1 p34.1-34.3. The distribution of signals was as follows: 1 chromatid (6 cells), two chromatids (14 cells) and three chromatids (5 cells). The mouse tssk 3b cDNA homologue mapped to the syntenic region in chromosome 4, band E.

### Example 2

### Cloning of the full length cDNA of the human homologues of the tssk kinase family and a partial cDNA sequence from the tssk substrate.

Using the predicted mouse tssk 1 and 2 amino acid sequences for the purposes of searching the gene bank, a genome sequence of both mouse tssk 1 and tssk2 was obtained. These genes mapped to chromosome 5 and 22 respectively and are intronless. The sequences corresponding to the 3' end and 5' end of the coding region were used to design sense and antisense primers respectively. The antisense primers were used in 5' RACE and the sense primer in 3' RACE using human testis marathon ready cDNA (Clontech, Inc.) as the template in order to ultimately obtain a full length sequence of both human kinase homologs. The amplified cDNA fragments were cloned using the TOPO TA cloning kit (Invitrogen) and sequenced. To amplify the full length human tssk kinases cDNA, the 5' end of the cDNA sequences obtained following 5' RACE were used to design a 5' sense primer. The 3' end of the cDNA sequences obtained following 3' RACE were used to design antisense primers. These two pairs of primers were then used to amplify human tssk1 and 2 from the same library. The amplified sequences 1.3 kb (tsskl) and of 1.2 kb (tssk 2) were subcloned and sequenced. The translated human homologues of the three members of the tssk kinase family are provided as SEQ ID NOS: 4-6, respectively.

To analyze the specificity of expression, commercial blots depicting several human tissues were performed using random primed-labeled probes from the full length cDNA of the human tssk 1 and 2. In addition a random prime-labeled probe from a partial 800 base pair sequence was used to determine the tissue distribution of the tssk substrate. Northern blots were performed using a commercially available human tissue blot (Clonetech).

Northern blots reveal that tssk 1, 2 and tssk substrate are testis specific mRNAs. These same blots were stripped to the tssk probes and reprobed with a beta actin sequence, confirming that each of the lanes was equally loaded with RNA. To further analyze the specificity of expression, the same probes were used to perform dot blots using commercially available mRNA arrays from 76 different human tissues (using the Multiple Tissue Expression (MTE™) Array from Clonetech, Cat # 7775-1. The only signal obtained in each of the probed MTEs is in the grid containing testis RNAs. This experiment confirmed that these messages are testis specific in human. In addition, Kueng et al. (1997) demonstrated that tssk 1 and 2 are postmeiotically expressed in mouse germ cells and that these messages are not present in other 11 tissues.

### Example 3

### Immunolocalization and immunoblotting experiments

In order to determine if the tssk 1,2 and 3 kinases and their substrate are present in the testis and/or mature sperm specific antibodies against the recombinant proteins will be generated. Alternatively, anti-peptide antibodies against specific peptides designed from the predicted amino acid sequence of each cDNA will be made. The specificity of each antibody generated will be tested against the recombinant tssk 1,2 and 3. It is expected that the anti-peptide antibodies designed against specific amino acid sequences of each protein will be specific.

Antibodies made against tssk kinases and against the tssk substrate will be used to analyze for the presence of these kinases in other tissues. For this purpose, Clontech protein MedleysTM of different tissues such as brain, heart, kidney, liver, lung, ovary, placenta, skeletal muscle and spleen will be tested by western blot using the anti tssk and anti tssk substrate antibodies. Since mRNAs coding for tssk 1,2 and 3 are only present in the testis, a similar protein distribution is expected. Since human homologues of tssk kinases have more than 80 % homology when compared with their mouse counterparts, it is expected that antisera against the human recombinant tssk kinases recognize the mouse homologues as well. Thus, the antibodies made against the human tssk kinases will be also tested in mouse tissues.

To generate polyclonal antibodies against the purified recombinant protein. Rats and rabbits will be used for production of antibodies. Protocol as described by Mandal et al. (1999) will be followed for this purpose. Antibody titers will be monitored by ELISA and specificity of the antibody will be checked by SDS-PAGE and Western blotting analysis.

### Sperm preparation.

In order to perform immunolocalization and immunoblotting experiments human sperm will be collected from healthy donors and purified using Percoll (Pharmacia Biotech, Upsala, Sweden) density gradient centrifugation as previously described (Naaby-Hansen et al., 1997). Sperm will be then resuspended to a final concentration of 2 x 10⁷ cells/ml.

### SDS-PAGE and immunoblotting.

Sperm and other cell types from different tissues will be pelleted by centrifugation, washed in 1 ml of phosphate buffered saline (PBS), resuspended in sample buffer (Laemmli, 1970) without mercaptoethanol and boiled for 5 min. After centrifuging, the supernatant will be saved, 2-mercaptoethanol will be added to a final concentration of 5 %, boiled for 5 min., and then subjected to 10 % SDS-PAGE. Protein concentration will be determined by ABC kit from Pierce. Electrophoretic transfer of proteins to Immobilon P and immunodetection will be carried out as previously described (Kalab et al., 1994). Gels will be stained either with silver, coomasie blue or will be transferred to immobilon PVDF (Millipore) and probed with the anti recombinant antibodies.

### Immunofluorescence.

To determine the intracellular location of tssk 1, 2 and 3, the specific antibodies against the soluble enzyme will be used in immunofluorescence experiments and immunoelectromicroscopy of human testicular tissue and human sperm. In addition, if the antibodies recognize the mouse antigens, the localization will be explored by immunofluorescence in mouse germ cells and sperm.

Sperm will be treated in the appropriate experimental conditions, fixed in suspension with a solution of 3 % (w/v) paraformaldehyde-0.05 % (v/v) glutaraldehyde in PBS for 1 h, washed in PBS at 37 C, and then permeabilized with 0.1 % (v/v) Triton X-100 in PBS at 37 C for 10 min. The sperm will then be washed in PBS and incubated overnight with serial dilutions (5, 10, 50 and 100) of the appropriate antibody as previously described (Visconti et al., 1996). After washing the sperm with PBS, they will be incubated with FITC-coupled goat anti mouse IgG and then attached to poly-lysine-coated microscope slides. Following 3 X washes with PBS, the slides will be mounted with fluoromont and fluorescence will be assessed. Testicular samples obtained from testicular biopsies will be processed as previosly described (Westbrook et al., 2000).

### Example 4

### Expression of recombinant tssk protein

Since many kinases have been expressed as active molecule in *E coli* (Bodenbach et al., 1994; Letwin et al., 1992). Advantage will be taken of *E coli*'s relatively simple, easy- to scale-up. Open reading frames of TSSK1, 2, 3 will be amplified and fused with his tag in pET28b vector (Novagen). The plasmid will be transformed into BL21 DE3 or other appropriate host strain. Recombinant protein production will be induced by addition of IPTG to 1mM final in the cultural medium. Recombinant protein will be purified from *E coli* lysate using Ni-NTA column under native condition. To facilitate crystal formation, highly purified protein is optimal. To purify the protein preparation from above, preparative electrophoresis using PrepCell (BIO-RAD) will be employed. Kinase assay will be conducted before proceeding to crystallography.

To produce recombinant testicular tssk kinases and tssk substrate in bacteria, expression constructs of tssk 1, 2 and 3 as well as for the tssk substrate will be made. The coding region of each of these proteins will be subcloned in the pET28b expression vector that contains at the C-terminus 6 residues of His-tag. In particular, the constructs will be made using complete ORF. primers that are designed to create an NdeI site at the 5' end and an XhoI site at the 3' end. The amplified products will be ligated into the NdeI-XhoI sites of pET28b expression vector. Since the recombinant protein will be fused with the 6 histidine residues of the expression vector the expressed protein will be purified using Ni-Histidine bind resin affinity chromatography. Once purified, kinase activity will be evaluated with the tssk substrate to make sure that the enzyme has folded correctly.

TSSK 2 protein was successfully expressed in E. coli and purified. To express tssk 2, the open reading frame was subcloned into a pET28b expression vector carrying a His-tag. Recombinant protein was produced following induction with IPTG. Bacterially expressed and partially purified tssk2 was further purified by preparative gel electrophoresis using a PrepCell from Biorad. The fractions were collected and analyzed by SDS-PAGE. TSSK substrate was expressed and purified similarly. These purified proteins were used to produce rat polyclonal antibodies using standard techniques.

Since multiple kinases have the property to become autophosphorylated *in vivo* and *in vitro*, purified bacterially expressed recombinant tssk 2 was assayed for autophosphorylation. The experiment was conducted in the presence of 40 µM ATP (1µCi of [³²P] ATP), 10 mM Mg²⁺, phosphatases inhibitors such as p-nitro-phenyl phosphate and glycerol phosphate and proteases inhibitors (leupeptin and aprotinin 10 µg/ml), the assay was stopped with sample buffer and tssk 2 was separated in 10 % PAGE. Auto radiography of the dried gel showed that recombinant tssk 2 incorporated ³²P, suggesting that the bacterially expressed tssk 2 folded correctly for phosphorylation to occur and could be used for structural studies

Since not all bacterial expressed recombinant proteins are folded correctly, a similar approach will be taken to express tssk 1, 2 and 3 in yeasts. In order to express the tssk kinases and substrate in yeasts, the kinases will be subcloned in pPICZαB vector from Invitrogen (Carlsbad, CA) and will be expressed in *Pichia pastoris* as secreted protein as well as an intracellular protein. These constructs also have a C-terminal His tag that will allow an easy purification of the recombinant proteins either from the culture media or from the extracted cells.

### Example 5

### Tssk 2 interacts with tsks in a yeast two-hybrid system

To demonstrate protein-protein interaction between tssk 2 and tsks a two yeast hybrid system was utilized, In this experiment, a bait gene (tssk 2) was first transformed into the reporter strain as a fusion to the GAL4 DNA binding domain (DNA-BD). A second plasmid that expressed tsks as fusion to the GAL 4 activation domain (AD) was also introduced into the AH109 reporter strain. Western blots were performed to confirm expression of fusion proteins in yeast. Interaction between tssk 2 and tsks was observed to promote transcription of the Histidine (HIS) gene and allow for the growth of yeast in His-free medium. Similarly, cotransfection of yeast cells with a first construct expressing a p53 fused to the GAL4 DNA binding domain and a second construct expressing the SV40 Large T antigen fused to the GAL 4 activation domain (AD) demonstrated that p53 and the SV40 Large T antigen interacted and promoted His gene activation. This interaction was used as a positive control. In contrast, fusion proteins of TSSK2 and p53 with the DNA-BD did not promote growing when cotransfected with the GAL-AD plasmid. Neither GAL-AD alone nor the fusion proteins between GAL-AD and TSKS or GAL-AD and SV40 Large T antigen had the ability to activate transcription of the His gene. This experiment demonstrated that tssk 2 and the substrate tsks are able to interact, suggesting that the human homologues of these proteins behave in a similar way to their mouse counterparts.

To investigate whether tssk 2 and tsks also interact *in vivo,* capacitated and non capacitated sperm will be extracted with Triton X 100 in conditions that protect protein-protein interaction and then immunoprecipitation will be conducted with specific antibodies. The interaction will be assayed by Western blots with the other antibody in a typical cross immunoprecipitation experiment. Since proteins that interact should co-localized, double labeling immunofluorescence will be conducted and co-localization investigated. Although rabbit antibodies have not yet been obtained for the human tssks, rat anti tssk 2 and rat anti tsks antibodies have been previosly obtained and therefore there is no reason to anticipate any difficulties in obtaining antibodies to the human tssks.

### Example 6

### Isolation of the Crystal Structure of tssk 1, 2,3 and the tssk substrate

Two strategies will be followed for the rationale design of tssk-specific inhibitors. First, an *in vitro* kinase assay compatible with High Throughput screening will be developed. Second, crystal structure of tssk kinases alone or complexed with their substrate will be obtained.

For structural studies using X-ray crystallography, it is necessary to obtain approximately 5 mg of the active kinases. This amount of protein is generally a suitable quantity for initial crystallization trials. Initial trials will be performed on the recombinant intact protein; this is a common crystallization technique that has proven to be successful in many cases, including several structural studies of enzymes. Prior to crystallization screening, the expressed fragment would be checked for a suitable level of enzymatic activity as well as for purity, homogeneity and solubility. Several commercially available crystallization screening kits, used in conjunction with the hanging drop vapor diffusion method, provide the standard first step in the search for crystal growth conditions. Crystallization screening of the fragment in the presence of catalytically required metal ions, substrates and/or inhibitors will be carried out simultaneously with the screen of the apoenzyme.

Crystals will be obtained at 21°C by equilibrating sitting drops. For cryodiffraction experiments, the crystals will be transferred to a similarly buffered solution through three solutions with intermediate concentrations of these reagents. For diffraction experiments at room temperature and lower resolution, Cu Kα radiation from rotating anode X-ray generators will be used. Final structure will be established after preliminary refinement, model improvement and further improvement.

In the case of the tssk kinase family, crystallization in the presence of their substrate will be attempted in order to establish a molecular basis of the kinase activity. -Once initial crystallization conditions are obtained, they will be optimized in order to obtain crystals that diffract to at least 3.0 Å resolution. Direct phasing of the structure via the MAD (multiple wavelength anomalous dispersion) and MIR (multiple isomorphous replacement) techniques would be carried out simultaneously, to assure success. Semi-automated model-building and refinement techniques for phased structures would be utilized to achieve rapid structural results. Once a structure is obtained, the results will be analyzed with a view to understanding the unique role of the tssk kinase family in spermatogenesis and/or sperm function. An ultimate goal of these studies would be the use of the structure as a template for the design of specific inhibitors of tssk 1, 2 and/or 3, which may prove useful as contraceptives.

### Example 7

### Development of a tssk-specific kinase assay.

The design of a specific kinase assay for the tssk family is advantageous from different points of view. First, an kinase assay will allow for the characterization of the kinetic properties of these enzymes such as the Km for ATP, divalent cation and substrate. Second, since only active enzymes are suitable for crystallographic studies, a kinase assay will confirm the folding of the recombinant proteins. Third, it is desireable to develop an *in vitro* kinase assay at the lab scale to be the basis to a kinase assay appropriate for high throughput screening of tssk-specific inhibitors.

Development of assays to measure tssk kinase activity will follow general characteristics of kinase assays such as presence of substrate, [γ32P]ATP, Mg²⁺ and/or Mn²⁺ and phosphatases inhibitors. First however a source of tssk kinase must be provided. The tssk kinase will be generated by recombinantly expressing these proteins and purifying the expressed kinase.

### Kinase assay in mammalian cell extracts.

ORFs of the respective human tssk kinases will be subcloned in a pCMV-HA epitope-tagged mammalian expression vector (Clontech cat # K6003-1). Similarly, the ORF of the tssk substrate will be subcloned in a pCMV-Myc mammalian expression vector (Clontech, same as above). Each of the three HA-kinases will be coexpressed with the cMyc-tssk substrate in three separate COS cell lines for each of the respective human tssks. Coexpression will be validated performing immunofluorescence with the respective antitag antibodies. Antibodies against HA and Myc are available from Clontech. Since anti c-Myc is a mouse monoclonal and anti HA is a rabbit polyclonal, it will be possible to analyze whether both the kinase and the substrate were coexpressed in the same cells.

Proteins will be then extracted with 1 % Triton and immunoprecipitation will be performed using anti HA-tag antibodies. Alternatively, anti tssk-kinase / anti-tssk substrate antibodies could be used to immunoprecipitate the tssk kinases and tssk substrate. Typically the antibodies will be linked to a solid support such as a sepharose bead to assist in the isolation of the target ligand. After precipitation with Protein A sepharose, the pellet will be assayed for kinase activity using different concentrations of ATP (10 µM, 100 µM and 1 mM), Mg²⁺ or Mn²⁺ (100 µM, 1 mM and 10 mM) and 1 µCi of [γ32P] ATP. The phosphorylated protein will be evaluated following SDS-PAGE separation and autoradiography. The evaluation of kinase activity after immunoprecipitation is a frequently used method that allow for specific measurement of the activity of one particular kinase (Coso et al., 1995, Cell 81, 1137-46; Moos et al., 1995, Biol Reprod 53, 692-9). Since Kueng et al. (1997) has successfully detected the phosphorylation of tssk substrate after immunoprecipitation of tssk 1 and 2, it is expected that it will be possible to measure kinase activity of tssk kinases after coexpression in a mammalian system.

### In vitro kinase assay.

Each purified enzyme will be mixed with different concentrations of ATP (10 µM, 100 µM and 1 mM), Mg²⁺ or Mn²⁺ (100 µM, 1 mM and 10 mM), 1 µCi of [γ32P] ATP and different concentrations of the purified tssk substrate (1, 10 and 100 ng/assay). Phosphorylation will be evaluated following SDS-PAGE separation and autoradiography. It is expected that if the proteins are correctly folded the phosphorylation of the substrate will be easily detected with this methodology.

### Example 8

### Generation of tssk antibodies

Purified tssk 2 and tsks were used to produce rat polyclonal antibodies. Antisera against recombinant human tssk 1, 2 and 3 as well as to the human tssk substrate will be employed to define their tissue distribution and subcellular localization at the protein level. The rat anti-tssk and anti-tsks antibodies recognized the recombinant protein and also proteins in sperm and testes with the predicted MW, suggesting that at least one member of the tssk family and their substrate (tsks) are present in sperm. These antibodies were then used to study the intracellular localization of these proteins in capacitated human sperm. Tssk 2 was observed to localized to the equatorial segment of human sperm. Tsks also localized to the equatorial segment Nevertheless anti-tsks antibody also recognize proteins present in the anterior head and in the tail. Immunolocalization of these molecules suggests that tssk 2 and tsks are present in similar region of the sperm at least in a fraction of the human sperm population. Control experiments were performed using rat pre immune serum of the respective antibody. Both Western blots and immunofluorescence were negative. Although the antibody against tssk 2 was produced against tssk 2, we can not discard that this antibody recognized other members of the tssk family since their sequences have high homology. However, discrimination between the three tssk isoenzymes, may be possible by generating antibodies against the C-terminal domain that is different between tssk 1 and 2 and is not present in tssk 3.

## Claims

1. A method for identifying antagonists of tssk kinase activity, the method comprising:
providing an *in vitro* kinase assay composition, said composition comprising a labeled source of phosphate, a tssk substrate comprising an amino acid sequence of SEQ ID NO: 8 and a tssk kinase, wherein the tssk kinase comprises SEQ ID NO: 6;
measuring the rate of phosphorylation of the substrate in the presence and the absence of a candidate inhibitory compound; and
identifying antagonist compounds that decrease tssk kinase activity to the kinase activity observed in the assays conducted in the absence of the antagonist compound.

2. The method of claim 1 wherein the labeled source of phosphate is [³²P] ATP.

3. The method of claim 1 wherein two or more tssk kinases are present in the kinase assay composition.

## Patentansprüche

1. Verfahren zur Identifizierung von Antagonisten der tssk-Kinase-Aktivität, wobei das Verfahren umfasst:
Bereitstellung einer *In-vitro*-Kinase-Assay-Zusammensetzung, wobei die Zusammensetzung eine markierte Phosphatquelle, ein tssk-Substrat, umfassend eine Aminosäuresequenz von SEQ ID NO: 8 und eine tssk-Kinase umfasst, wobei die tssk-Kinase SEQ ID NO: 6 umfasst;
Messung der Phosphorylierungsrate des Substrats in Gegenwart und Abwesenheit einer möglichen inhibitorischen Verbindung; und
Identifizierung von Antagonist-Verbindungen, die die tssk-Kinase-Aktivität auf die Kinase-Aktivität erniedrigen, die in unter Abwesenheit der Antagonist-Verbindung durchgeführten Assays beobachtet wurde.

2. Verfahren gemäß Anspruch 1, wobei die markierte Phosphatquelle [³²P] ATP ist.

3. Verfahren gemäß Anspruch 1, wobei zwei oder mehr tssk-Kinasen in der Kinase-Assay-Zusammensetzung vorhanden sind.

## Revendications

1. Une méthode pour l'identification des antagonistes de l'activité de la kinase tssk, ladite méthode comprenant:
la préparation d'une composition d'un essai kinase *in vitro,* ladite composition comprenant une source de phosphate marqué, un substrat pour le tssk comprenant une séquence d'un acide aminé d'après SEQ ID NO:8 et une kinase tssk, la kinase tssk comprenant la SEQ ID NO:6;
le mesurage du taux de phosphorylation du substrat en présence et en absence d'un candidat du composé inhibiteur; et
l'identification des composés antagonistes qui deminués l'activité de la kinase tssk à l'activité de kinase qui est observé dans les essais effectués en absence du composé d'antagoniste.

2. La méthode selon la revendication 1 dans laquelle la source de phosphate marqué est [³²P] ATP.

3. La méthode selon la revendication 1 dans laquelle deux ou plus de deux kinases tssk sont presentes dans la composition d'essai kinase.
